# EUROPÄISCHE PATENTSCHRIFT

(11) **EP 1 452 202 B1**
(45) Veröffentlichungstag und Bekanntmachung des Hinweises auf die Patenterteilung: **03.08.2005**
(21) Anmeldenummer: 04000219.8
(22) Anmeldetag: 08.01.2004
(51) Int. Cl.: A61M 39/28

(54) **Rollenklemme zum Regulieren der Durchflussmenge durch einen Schlauch**
Roller clamp for regulating the fluid flow through a tube
Pince à galet pour régler le débit dans un tube

(30) Priorität: 26.02.2003 DE 10308118
(43) Veröffentlichungstag der Anmeldung: 01.09.2004
(73) Patentinhaber: Forberg, Hans-Jürgen, 23758 Oldenburg (DE)
(72) Erfinder: Forberg, Hans-Jürgen, 23758 Oldenburg (DE)
(74) Vertreter: Vollmann, Heiko, Dipl.-Ing.

(56) Entgegenhaltungen:
- DE-C- 3 738 965
- US-A- 3 802 463
- US-A- 4 475 709
- US-A- 4 856 755
- US-A- 4 869 721

## Beschreibung

Die Erfindung geht aus von einer Rollenklemme zum Einstellen des Regulierquerschnittes eines in die Rollenklemme eingelegten Schlauches, der an einen medizinischen Flüssigkeitsbehälter anschließbar ist, wobei die Rollenklemme ein längliches, im Querschnitt u-förmiges Klemmgehäuse mit zwei Seitenwänden und einer diese verbindenden Stegwand sowie eine in diesen Seitenwänden längsverschieblich angeordnete, auf den in dem Gehäuse positionierten Schlauch einwirkenden Klemmrolle mit einem Ringfalz an ihrem einen Umfangseckbereich zur Bildung des Regulierquerschnittes des Schlauches unter Mitwirkung des Klemmgehäuses aufweist.

Mit Hilfe einer solchen Rollenklemme kann der Strömungsquerschnitt des Schlauches durch Verschieben der Klemmrolle in dem Klemmgehäuse verändert und so die Durchflussmenge eines flüssigen Mediums eingestellt werden.

Eine derartige Rollenklemme ist in der DE 37 38 965 C1 beschrieben. Diese Rollenklemme besteht aus einem länglichen, u-förmigen Gehäuse und aus einer darin in Längsrichtung geführt verschiebbaren Klemmrolle. Die Klemmrolle besitzt an ihrem einen Umfangseckbereich einen Ringfalz zur Aufnahme eines abgewinkelten Längsrandbereiches des flexiblen, zwischen der Klemmrolle und dem Steg des Gehäuses lageunveränderlich eingeklemmten Schlauches, wobei der Ringfalz mit der ihm gegenüberliegenden Seitenwand des Klemmgehäuses den jeweiligen Regulierbereich des Klemmgehäuses bildet. Diese Seitenwand ist mit einem vorstehenden Mittel in Form einer geneigt verlaufenden Leiste zum Miteinstellen des Regulierquerschnittes des Schlauches versehen, so dass der Regulierquerschnitt des Schlauches durch Verschieben der Rollenklemme in dem Klemmgehäuse vergrößert bzw. verkleinert wird.

Diese Rollenklemme hat sich in der Praxis bewährt, d. h. der jeweils eingestellte Regulierquerschnitt bleibt im Wesentlichen konstant erhalten. Da jedoch das Material des verwendeten Schlauches in seiner Weichheit unterschiedlich sein kann, besteht bei relativ weichem Schlauchmaterial die Gefahr, dass beim Rückverschieben der Klemmrolle, d. h. wenn der Regulierquerschnitt des Schlauches vergrößert werden soll, oder wenn beim genauen Einstellen des Regulierquerschnittes eine Rückbewegung der Klemmrolle erfolgt, Schlauchmaterial in dem oberen Bereich des leistenartigen Einstellmittels fest eingeklemmt wird. Daraus folgt, dass in einem solchen Fall der gewünschte Regulierquerschnitt des Schlauches nicht genau eingestellt werden kann und/oder dass der Schlauch entlang der Einstellstrecke seines Regulierquerschnittes in dem Klemmgehäuse beschädigt wird.

Eine weitere Rollenklemme ist aus der DE 22 42 539 C2 bekannt. Sie ist an einem Längsrand des Steges des Klemmgehäuses mit einer sich insbesondere in der Breite verjüngenden, kanalförmigen Aussparung versehen, die eine solche Tiefe aufweist, dass sich der den Regulierquerschnitt des Schlauches bildende Abschnitt dieses Schlauches unter Abwinkelung frei in die jeweilige Querschnittsstelle der Aussparung hineinzwängen kann. Der Querschnitt der Aussparung verändert sich kontinuierlich in der Längsrichtung, so dass bei Verschiebung der Klemmrolle der entsprechende Regulierquerschnitt des Schlauches eingestellt wird. An dem anderen Längsrandbereich des Gehäusesteges, der der kanalförmigen Aussparung abgekehrt, ist eine Fixierungserhöhung vorgesehen, die den zwischen der Klemmenrolle und dem Steg eingeklemmten und an dieser Stelle nicht durchströmbaren Schlauch allseits fixiert festhält. Durch diese Ausbildung kann der Schlauch zur Erzielung eines genauen und konstant bleibenden Regulierquerschnittes entlang der Regulierstrecke des Klemmgehäuses nicht optimal eingestellt werden.

Die Aufgabe der Erfindung besteht in der Verbesserung einer Rollenklemme der einleitend angeführten Art dahingehend, dass eine Beschädigung des Schlauches entlang seiner Einstellstrecke in dem Klemmgehäuse vermieden und eine optimale Einstellung des Schlauches in dem Klemmgehäuse zur Erzielung eines genauen und konstanten Regulierquerschnittes des Schlauches entlang seiner Einstellstrecke gewährleistet ist.

Die Lösung der Aufgabe ist in dem Patentanspruch 1 angegeben.

Die mit der erfindungsgemäßen Lösung erzielbaren Vorteile bestehen insbesondere darin, dass beim Verstellen der Klemmrolle, vorzugsweise beim Rückverstellen der Klemmrolle in dem Klemmgehäuse, d. h. ein Verstellen der Klemmrolle in diejenige Stellung, in welcher der Regulierquerschnitt des in der Rollenklemme befindlichen Schlauches größer wird, ein Beschädigen des Schlauches entlang der Einstellstrecke der Klemmrolle insbesondere bei sehr weichem Schlauchmaterial vermieden ist. Derjenige Schlauchlängsbereich, welcher dem Falz der Klemmrolle und damit dem Regulierquerschnitt des Schlauches zugekehrt ist, wird beim Verstellen der Klemmrolle durch die erfindungsgemäß auf der anderen Seite des Klemmgehäuses vorgesehene Seitenführungsfläche schonend dem Regulierungsvorgang unterworfen, so dass der Schlauch im Bereich seines Regulierquerschnittes keinen schädigenden Quetschungen während der Verschiebung der Klemmrolle ausgesetzt ist. Die Seitenführungsfläche bewirkt ein Vorschieben des Schlauches quer zu seiner Längsrichtung unter der Klemmenrolle in Richtung des Regulierquerschnitts. Somit wird auch bei weichen Schlauchmaterialien eine optimale Lage des Schlauches in seiner jeweiligen Klemm- und Regulierposition erreicht, wobei eine sehr genaue und konstante Einstellung des Regulierquerschnittes gewährleistet ist. Durch die Querschiebung des Schlauches wird außerdem ein größerer Regulierungsquerschnitt des Schlauches erreicht, der für mit Ballaststoffen versehene Nährlösungen vorteilhaft ist.

In einer vorteilhaften Ausgestaltung der erfindungsgemäßen Seitenführungsfläche des Klemmgehäuses ist diese an einem leistenförmigen Vorsprung vorgesehen, der vorzugsweise mit der Seitenwand und der Stegwand des Klemmgehäuses einstückig ausgebildet ist. Durch diese Ausbildung lässt sich die Seitenführungsfläche auf einfache Weise beim Spritzgießen der Rollenklemme herstellen.

In weiterer vorteilhafter Ausgestaltung verläuft die Seitenführungsfläche des Klemmgehäuses in einem stumpfen Winkel zur Stegwand des Klemmgehäuses. Durch diese Ausbildung wird der entsprechende Seitenbereich des Schlauches beim Verschieben der Klemmrolle besonders schonend in seine Querlage in dem Klemmgehäuse gebracht.

Die erfindungsgemäße Seitenführungsfläche des Klemmgehäuses ist insbesondere auch dann von besonderem Vorteil, wenn als Klemmhilfsmittel für die Einstellung des Regulierquerschnittes des Schlauches eine Einstellnut in derjenigen Seitenwand des Klemmgehäuses vorgesehen ist, die dem Ringfalz der Klemmrolle zugekehrt ist. Die erfindungsgemäße Seitenführungsfläche bewirkt eine beschädigungsfreie Einbringung und Führung des den Regulierquerschnitt bildenden Schlauchbereiches in die bzw. in der Einstellnut entlang der gesamten Einstellstrecke für den Regulierquerschnitt. Somit ist auch hier bei insbesondere weichen Schlauchmaterialien gewährleistet, dass der Schlauch entlang seiner Regulierstrecke während der Einstellung des Regulierquerschnittes nicht beschädigt wird.

In einer anderen vorteilhaften Ausbildung verläuft die Bodenfläche der Einstellnut zur Längsmittelebene des Klemmgehäuses ebenfalls geneigt. In diesem Zusammenhang besteht eine besondere Ausbildung darin, dass die Bodenfläche der Einstellnut zu der erfindungsgemäßen Führungsfläche des Klemmgehäuses äquidistant verläuft. Eine hierfür besonders einfache Ausbildung besteht darin, dass die Bodenfläche der Einstellnut und die erfindungsgemäße Seitenführungsfläche zueinander parallel verlaufen.

Die Erfindung ist nachstehend anhand eines in den anliegenden Zeichnungen dargestellten Ausführungsbeispieles näher erläutert. Es zeigen:
- Fig. 1: einen ersten Längsschnitt durch das Ausführungsbeispiel nach der Linie D - D in Fig. 2,
- Fig. 2: einen zweiten Längsschnitt durch die Rollenklemme nach der Linie A - A in Fig. 1,
- Fig. 3: einen Querschnitt nach der Linie B - B in Fig. 1,
- Fig. 4: einen weiteren Querschnitt nach der Linie C - C in Fig. 1.

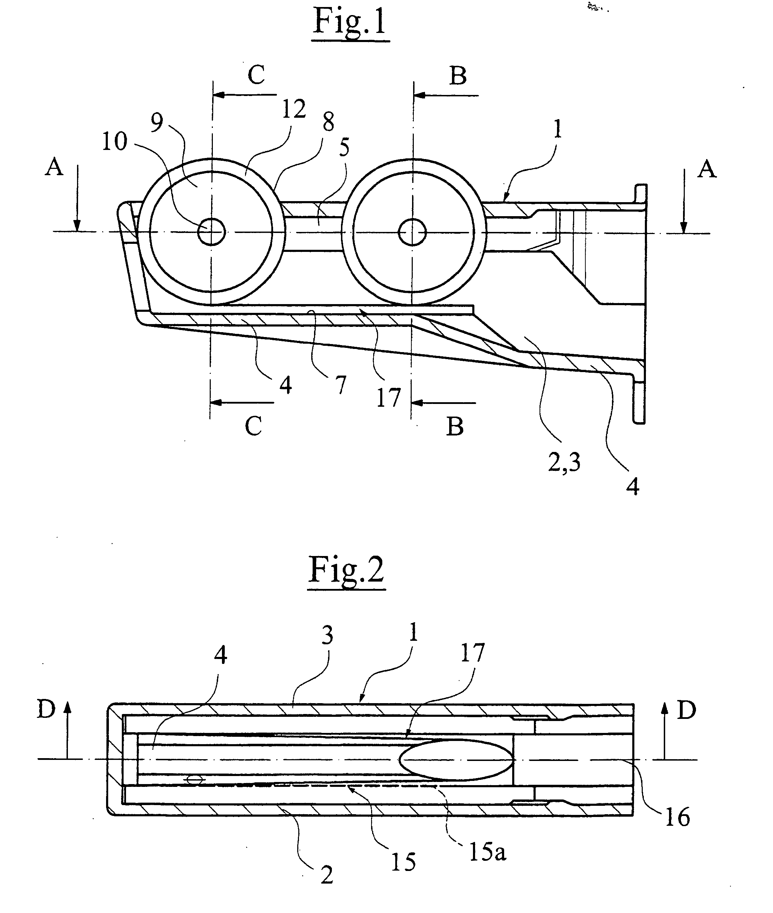

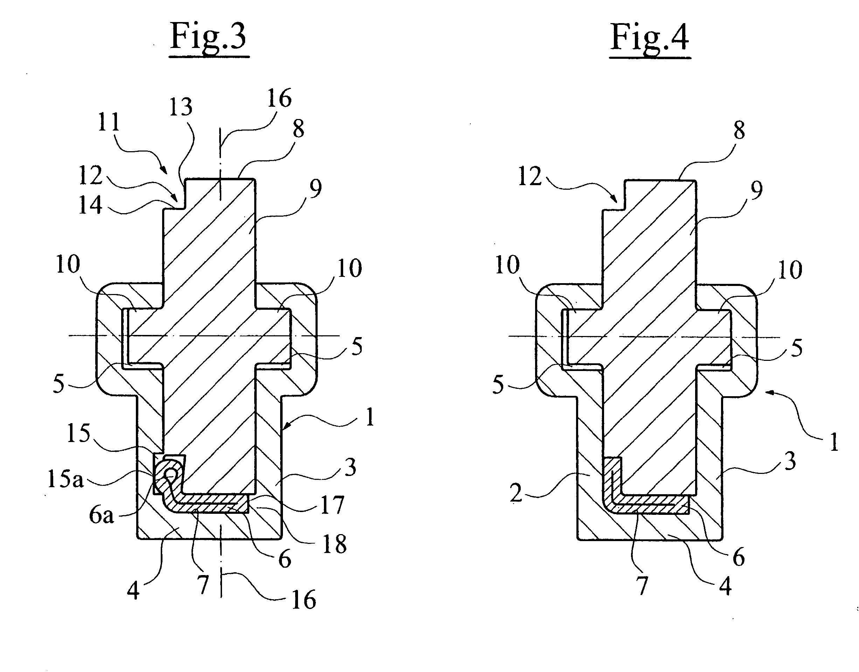

Die Rollenklemme besteht gemäß Fig. 1 in üblicher Weise aus einem länglichen, vorzugsweise im Spritzgießverfahren hergestellten, u-förmigen Kunststoff-Klemmgehäuse 1 mit zwei Seitenwänden 2 und 3 sowie mit einer Stegwand 4. In den Seitenwänden 2 und 3 sind zu der Stegwand 4 parallel verlaufende Führungsnuten 5 eingearbeitet, die in bekannter Weise zu dem einen Ende des beidendig offenen Klemmgehäuses 1 hin offen sind, um die Klemmrolle in dem Gehäuse 1 anordnen zu können. Die Innenfläche 7 der Stegwand 4 bildet eine Klemmfläche für einen flexiblen Schlauch 6, der nur in den Fig. 3 und 4 dargestellt und dort von der Umfangfläche 8 einer Klemmrolle 9 eingeklemmt wird. Die Klemmrolle 9 weist in ihrem Zentrum beidseitig angeordnete Achszapfen 10 auf, die in die Führungsnuten 5 eingreifen. Die Umfangsfläche 8 der Klemmrolle 9 ist beispielsweise durch Rändelung griffig gestaltet und liegt der Klemmfläche 7 in einem Abstand gegenüber, der aufgrund des Spiels zwischen den Zapfen 10 und den zugehörigen Nuten 5 geringfügig kleiner ist als die doppelte Wandstärke des flexiblen Schlauches 6. Gemäß den Figuren 3 und 4 wird zwischen der Umfangsfläche 8 der Klemmrolle 9 und der Klemmfläche 7 der Gehäusestegwand 4 ein erster Querbereich des Schlauches 6 eingeklemmt, derart, dass in diesem Bereich des Schlauches kein Strömungsquerschnitt ausgebildet, der Schlauch aber in dem Gehäuse 1 schonend, d. h. zerstörungsfrei und querbeweglich, gehalten wird.

Wie es am Besten in den Figuren 3 und 4 zu erkennen ist, weist die Klemmrolle 9 an ihrem einen Umfangseckbereich 11 einen konzentrischen Ringfalz 12 mit einer radialen Anlagefläche 13 und mit einer achsparallelen Anlagefläche 14 auf. Vorzugsweise ist die radiale Anlagefläche 12 zurückspringend ausgebildet, so dass sie in Bezug auf die Klemmfläche 7 der Stegwand 4 geneigt verläuft. Der Verlauf der Neigung ist in den Figuren 3 und 4 zu erkennen.

Die Anlageflächen 13 und 14 bilden eine erste Wandbegrenzung zur Ausbildung eines örtlichen Regulierquerschnittes 6a als übrigen Querbereich des Schlauches 6. Man erkennt, dass der jeweils diesen Regulierquerschnitt bildende Bereich des Schlauches gemäß den Figuren 3 und 4 abgewinkelt in den Ringfalz 11 der Klemmrolle 9 eingreift.

Als zweite und letzte Wandbegrenzung zur Ausbildung des jeweiligen Regulierquerschnittes 6a des Schlauches 6 ist die eine, der Anlagefläche 13 des Ringfalzes 12 der Rollenklemme 9 gegenüberliegende Seitenwand 2 der Klemmgehäuses 1 vorgesehen. Vorzugsweise ist diese Seitenwand 2 gemäß Fig. 3 vorteilhaft mit einer Einstellnut 15 versehen, deren Bodenfläche 15a querschnittsvermindernd ausgebildet ist, so dass diese den jeweils erforderlichen Regulierquerschnitt 6a des Schlauches 6 beim Verschieben der Klemmrolle 9 einstellt. Um die Querschnittsveränderung des Regulierquerschnittes zu erreichen, verläuft die Bodenfläche 15a der Einstellnut 15 geneigt zur Längsmittelebene 16 des Klemmgehäuses 1, wie dies deutlich in Fig. 2 zu erkennen ist. Der geneigte Verlauf der Bodenfläche 15a selbst kann eben oder leicht konkav oder leicht konvex gekrümmt ausgebildet sein, was von dem jeweiligen Anwendungsfall abhängt. Somit bildet die Nut 15 bzw. deren Bodenfläche 15a ein Einstellmittel, das bei Verschiebung der Klemmrolle 9 eine Veränderung der Größe des Regulierquerschnittes 6a mit bewirkt.

Wie Fig. 3 zeigt, ist die Einstellnut 15 im Querschnitt rechteckig ausgebildet, derart, dass ihre Bodenfläche 15a zur Innenfläche 7 der Stegwand 4 im rechten Winkel verläuft. Alternativ kann auch so vorgegangen werden, dass die Bodenfläche 15a mit der genannten Innenfläche 7 einen stumpfen Winkel (nicht dargestellt) einschließt. Hierdurch wird ein zu starkes Einknicken der äußeren Schlauchwand im Übergangsbereich zwischen dem Regulierquerschnitt 6a und dem übrigen Klemmquerschnitt des Schlauches 6 vermieden, so dass also in diesem Übergangsbereich eine schonende Verformung des Schlauches gegeben ist.

Wie es am Besten in den Figuren 2 und 3 zu sehen ist, ist das Klemmgehäuse 1 in seinem Übergangsbereich von der anderen Seitenwand 3, die dem Ringfalz 12 der Klemmrolle 9 abgekehrt ist, zu der Stegwand 4 des Klemmgehäuses mit einer inneren, länglichen Seitenführungsfläche 17 für den zwischen der Stegwand 7 und der Umfangsfläche 8 der Klemmrolle 9 gehaltenen Schlauch 6 versehen. Wie insbesondere Fig. 2 zeigt, verläuft die Seitenführungsfläche 17 allmählich in das Gehäuseinnere vorspringend, und zwar derart, dass sie zur Längsmittelebene 16 des Gehäuses 1 geneigt ist. Der vorspringende Verlauf der Seitenführungsfläche ist in Richtung zur Seitenwand 2 des Gehäuses 1 gerichtet, so dass der leicht geklemmte Bereich des Schlauches 6 quer zur Längsrichtung des Klemmgehäuses 1 geführt bewegt wird, wie es in Fig. 2 und bei einem Vergleich der Figuren 3 und 4 zu erkennen ist.

Die Seitenführungsfläche 17 des Klemmgehäuses 1 ist vorzugsweise an einem leistenförmigen Vorsprung 18 vorgesehen. Dieser Vorsprung 18 ist mit der Seitenwand 3 und der Stegwand 4 des Klemmgehäuses 1 vorteilhaft einstückig ausgebildet, so dass dieser Vorsprung bei der Gießherstellung des Gehäuses 1 auf einfache Weise mitgebildet wird.

Die Seitenführungsfläche 17 kann, wie in Fig. 3 dargestellt, mit der Stegwand 4 des Klemmgehäuses 1 einen rechten Winkel bilden. Es ist jedoch auch möglich, dass die Seitenführungsfläche mit dieser Stegwand einen stumpfen Winkel einschließt, was die Seitenführung des Schlauches 6 verbessert. Bezug nehmend auf Fig. 2 ist zu erkennen, dass die geneigt verlaufende Seitenführungsfläche 17 selbst eben ausgebildet ist. Ebenfalls ist gemäß Fig. 2 die Bodenfläche 15a der Einstellnut 15 eben ausgebildet. Beide Flächen 15a und 17 verlaufen des Weiteren so, das zwischen ihnen beiden entlang ihrer Länge ein äquidistanter Abstand besteht.

Im dargestellten Fall nach Fig. 2 verlaufen die Bodenfläche 15a und die Seitenführungsfläche 17 parallel zueinander. Ein äquidistanter Verlauf dieser beiden Flächen zueinander ist aber auch dann gegeben, wenn diese beiden Flächen gleichsinnig gekrümmt verlaufen. Eine solche Krümmung wird naturgemäß sehr schwach sein. Wenn beispielsweise die Seitenführungsfläche 17 schwach konvex gekrümmt verläuft, wird die Bodenfläche 15a schwach konkav gekrümmt verlaufen. Als weitere Alternative ist es möglich, dass, wenn die eine Fläche 15a oder 17 gerade bzw. eben verläuft, die jeweils andere Fläche schwach gekrümmt verläuft. In diesem Fall ist kein äquidistanter Verlauf gegeben.

Fig. 1 ist so zu verstehen, dass üblicherweise nur eine einzige Klemmrolle 9 vorgesehen ist. Die Stellung B - B in Fig. 1 bedeutet, dass sich diese Klemmrolle 9 in ihrer einen Endstellung befindet, in welcher der Regulierquerschnitt 6a des in die Rollenklemme eingelegten Schlauches am größten ist, wie dies in Fig. 3 dargestellt ist. In der anderen Endstellung der Klemmrolle 9, welche in Fig. 1 durch die Position C - C dargestellt ist, ist der Schlauch 6 vollständig geschlossen, d. h. er weist keinen eigentlichen Regulierquerschnitt mehr auf, was der Darstellung des Schlauches in Fig. 4 entspricht. Zwischen den beiden vorgenannten Endstelllungen der Klemmrolle 9 werden die jeweils gewünschten Zwischenregulierquerschnitte des Schlauches 6 durch Verschieben der Klemmrolle 9 bei eigener Drehung in den Führungsnuten 5 in üblicher Weise eingestellt.

## Patentansprüche

1. Rollenklemme zum Einstellen des Regulierquerschnittes eines Schlauches, der an einen medizinischen Flüssigkeitsbehälter anschließbar ist, umfassend ein längliches, im Querschnitt u-förmiges Klemmgehäuse (1) mit zwei Seitenwänden (2, 3) und einer diese verbindenden Stegwand (4) sowie eine in diesen Seitenwänden längsverschieblich angeordnete, auf den in dem Gehäuse positionierten Schlauch einwirkende Klemmrolle (9) mit einem Ringfalz (12) an ihrem einen Umfangseckbereich zur Bildung eines Regulierquerschnittes des Schlauches unter Mitwirkung des Klemmgehäuses (1), **dadurch gekennzeichnet, dass** das Klemmgehäuse (1) in seinem Übergangsbereich von der Stegwand zu derjenigen Seitenwand (3), die dem Ringfalz (12) der Klemmrolle (9) abgekehrt ist, eine innere, längliche Seitenführungsfläche (17) für den zwischen der Stegwand und der Klemmrolle gehaltenen Schlauch (6) aufweist, die in das Klemmgehäuse (1) vorsteht und zu dessen Längsmittelbene (16) geneigt verläuft.

2. Rollenklemme nach Anspruch 1, **dadurch gekennzeichnet, dass** die Seitenführungsfläche (17) des Klemmgehäuses (1) an einem leistenförmigen Vorsprung (18) vorgesehen ist.

3. Rollenklemme nach Anspruch 2, **dadurch gekennzeichnet, dass** der Vorsprung (18) mit der Seitenwand (3) und der Stegwand (4) des Klemmgehäuses (1) einstückig ausgebildet ist.

4. Rollenklemme nach Anspruch 1, 2 oder 3, **dadurch gekennzeichnet, dass** die Seitenführungsfläche (17) des Klemmgehäuses (1) in einem stumpfen Winkel zu der Stegwand (4) des Klemmgehäuses verläuft.

5. Rollenklemme nach einem der Ansprüche 1 bis 4, **dadurch gekennzeichnet, dass** der Seitenführungsfläche (17) des Klemmgehäuses (1) in derjenigen Seitenwand (2) dieses Gehäuses, die dem Ringfalz (12) der Klemmrolle (9) zugekehrt ist, eine Einstellnut (15) zugeordnet ist, deren Querschnitt sich entlang der Verschiebestrecke der Klemmrolle (9) zur Einstellung des jeweiligen Regulierquerschnittes (6a) des Schlauches (6) veränderlich ausgebildet ist.

6. Rollenklemme nach Anspruch 5, **dadurch gekennzeichnet, dass** die Bodenfläche (15a) der Einstellnut (15) zur Längsmittelebene (16) des Klemmgehäuses (1) geneigt verläuft.

7. Rollenklemme nach Anspruch 5 oder 6, **dadurch gekennzeichnet, dass** die Bodenfläche (15a) der Einstellnut (15) zu der Seitenführungsfläche (17) des Klemmgehäuses (1) äquidistant verläuft.

8. Rollenklemme nach Anspruch 5 oder 6, **dadurch gekennzeichnet, dass** die Bodenfläche (15a) der Einstellnut (15) zu der Seitenführungsfläche (17) des Klemmgehäuses (1) parallel oder im Wesentlichen parallel verläuft.

## Claims

1. A roller pincher for setting the regulation cross-section of a tubing which is connectable to a medical fluid container, comprising an elongate pinch housing (1) u-shaped in cross-section with two sidewalls (2, 3) and with a web wall (4) connecting these, as well as a pinch roller (9) which acts on the tubing positioned in the housing and is arranged longitudinally displaceable in these sidewalls, with an annular notch (12) on its one circumferential corner region for forming a regulation cross-section of the tubing in cooperation with the pinch housing (1), **characterised in that** the pinch housing (1) in its transition region from the web wall to that sidewall (3) which is distant to the annular notch (12) of the pinch roller (9) comprises an inner, elongate side guide surface (17) for the tubing (6) held between the web wall and the pinch roller, said side guide surface projecting into the pinch housing (1) and running inclined to its longitudinal middle plane (16).

2. A roller pincher according to claim 1, **characterised in that** the side guide surface (17) of the pinch housing (1) is provided on a ledge-like projection (18).

3. A roller pincher according to claim 2, **characterised in that** the projection (18) is formed as one piece with the sidewall (3) and the web wall (4) of the pinch housing (1).

4. A roller pincher according to claim 1, 2 or 3, **characterised in that** the side guide surface (17) of the pinch housing (1) runs at an obtuse angle to the web wall (4) of the pinch housing.

5. A roller pincher according to one of the claims 1 to 4, **characterised in that** an adjusting groove (15) is allocated to the side guide surface (17) of the pinch housing (1) **in that** sidewall (2) of this housing which faces the annular notch (12) of the pinch roller (9), whose cross-section is designed changing along the displacement path of the pinch roller (9) for setting the respective regulation cross-section (6a) of the tubing (6).

6. A roller pincher according to claim 5, **characterised in that** the base surface (15a) of the adjusting groove (15) runs inclined to the longitudinal middle plane (16) of the pinch housing (1).

7. A roller pincher according to claim 5 or 6, **characterised in that** the base surface (15a) of the adjusting groove (15) runs equidistant to the side guide surface (17) of the pinch housing (1).

8. A roller pincher according to claim 5 or 6, **characterised in that** the base surface (15a) of the adjusting groove (15) runs parallel or essentially parallel to the side guide surface (17) of the pinch housing (1).

## Revendications

1. Pince à galet pour le réglage de la section transversale régulatrice d'un tuyau souple qui peut être raccordé à un réservoir de liquide à usage médical, comprenant un corps de pince allongé (1) ayant la forme d'un U en coupe transversale et comportant deux parois latérales (2, 3) et une paroi centrale (4) reliant ces demières, ainsi qu'un galet de pincement (9) monté à coulissement longitudinal dans ces parois latérales et agissant sur le tuyau souple positionné dans le corps, lequel galet de pincement présente, au niveau de l'une de ses régions d'angle périphériques, une feuillure annulaire (12) destinée à la formation d'une section transversale régulatrice du tuyau souple en coopération avec le corps de pince (1), **caractérisée en ce que** le corps de pince (1) présente, dans sa région de transition de la paroi centrale à la paroi latérale (3) qui est située à l'opposé de la feuillure annulaire (12) du galet de pincement (9), une surface de guidage latéral intérieure (17), allongée, pour le tuyau souple (6) retenu entre la paroi centrale et le galet de pincement, laquelle est formée en saillie dans le corps de pince (1) et est inclinée par rapport au plan médian longitudinal (16) de celui-ci.

2. Pince à galet selon la revendication 1, **caractérisée en ce que** la surface de guidage latéral (17) du corps de pince (1) est prévue sur une partie saillante (18) en forme de baguette.

3. Pince à galet selon la revendication 2, **caractérisée en ce que** la partie saillante (18) est formée d'un seul tenant avec la paroi latérale (3) et la paroi centrale (4) du corps de pince (1).

4. Pince à galet selon la revendication 1, 2 ou 3, **caractérisée en ce que** la surface de guidage latéral (17) du corps de pince (1) s'étend sous un angle obtus par rapport à la paroi centrale (4) du corps de pince.

5. Pince à galet selon l'une des revendications 1 à 4, **caractérisée en ce qu'**à la surface de guidage latéral (17) du corps de pince (1) est associée, dans la paroi latérale (2) de ce corps, qui est tournée vers la feuillure annulaire (12) du galet de pincement (9), une rainure de réglage (15) dont la section transversale varie dans son développement le long du trajet de coulissement du galet de pincement (9), en vue du réglage de la section transversale régulatrice respective (6a) du tuyau souple (6).

6. Pince à galet selon la revendication 5, **caractérisée en ce que** la surface de fond (15a) de la rainure de réglage (15) est inclinée par rapport au plan médian longitudinal (16) du corps de pince (1).

7. Pince à galet selon la revendication 5 ou 6, **caractérisée en ce que** la surface de fond (15a) de la rainure de réglage (15) s'étend à équidistance de la surface de guidage latéral (17) du corps de pince (1).

8. Pince à galet selon la revendication 5 ou 6, **caractérisée en ce que** la surface de fond (15a) de la rainure de réglage (15) s'étend parallèlement ou sensiblement parallèlement à la surface de guidage latéral (17) du corps de pince (1).
